(19) **Europäisches Patentamt / European Patent Office / Office européen des brevets**

(11) **EP 4 426 253 B1**

(12) **EUROPEAN PATENT SPECIFICATION**

(45) Date of publication and mention
of the grant of the patent:
**17.09.2025 Bulletin 2025/38**

(21) Application number: **23762576.9**

(22) Date of filing: **06.07.2023**

(51) International Patent Classification (IPC):
**A61F 13/00** *(2024.01)* **A61L 15/26** *(2006.01)*
**A61L 15/44** *(2006.01)* **A61F 13/01** *(2024.01)*

(52) Cooperative Patent Classification (CPC):
(C-Sets available)
**A61L 15/44; A61F 13/00063; A61F 13/01017;
A61L 15/26;** A61L 2300/404 (Cont.)

(86) International application number:
**PCT/PL2023/050051**

(87) International publication number:
**WO 2024/010475 (11.01.2024 Gazette 2024/02)**

(54) **ABSORBENT MATERIAL MADE OF PGS, IN PARTICULAR DRESSING MATERIAL, CONTAINING AN ACTIVE SUBSTANCE, METHOD OF PGS ABSORBENT MATERIAL FABRICATION AND ITS USE**

WIRKSTOFFHALTIGES ABSORBIERENDES MATERIAL AUS PGS, INSBESONDERE VERBANDMATERIAL, VERFAHREN ZUR HERSTELLUNG DES ABSORBIERENDEN PGS-MATERIALS UND DESSEN VERWENDUNG

MATÉRIAU ABSORBANT CONSTITUÉ DE PGS, EN PARTICULIER MATÉRIAU DE PANSEMENT, CONTENANT UNE SUBSTANCE ACTIVE, PROCÉDÉ DE FABRICATION DE MATÉRIAU ABSORBANT EN PGS ET SON UTILISATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.07.2022 PL 44165622**

(43) Date of publication of application:
**11.09.2024 Bulletin 2024/37**

(73) Proprietor: **POLITECHNIKA WARSZAWSKA**
**00-661 Warszawa (PL)**

(72) Inventors:
• **GADOMSKA-GAJADHUR, Agnieszka**
**03-684 Warszawa (PL)**
• **WRZECIONEK, Michal**
**25-366 Kielce (PL)**
• **RUSKOWSKI, Pawel**
**05-200 Lipinki (PL)**
• **CIELOCH, Marta**
**01-360 Warszawa (PL)**

(74) Representative: **JWP Patent & Trademark Attorneys**
**ul. Minska 75**
**03-828 Warszawa (PL)**

(56) References cited:
**EP-B1- 1 448 656**

• YOU Z ET AL: "A functionalizable polyester with free hydroxyl groups and tunable physiochemical and biological properties", BIOMATERIALS, ELSEVIER, AMSTERDAM, NL, vol. 31, no. 12, 1 April 2010 (2010-04-01), pages 3129 - 3138, XP027575354, ISSN: 0142-9612, [retrieved on 20100209]

- **SEYED AHMAD AYATI NAJAFABADI ET AL: "Evaluation of sustained ciprofloxacin release of biodegradable electrospun gelatin/poly(glycerol sebacate) mat membranes for wound dressing applications", ASIA-PACIFIC JOURNAL OF CHEMICAL ENGINEERING, JOHN WILEY & SONS LTD, US, vol. 13, no. 6, 8 October 2018 (2018-10-08), pages n/a, XP072441712, ISSN: 1932-2135, DOI: 10.1002/APJ.2255**

(52) Cooperative Patent Classification (CPC): (Cont.)

C-Sets
**A61L 15/26, C08L 67/04**

**Description**

The present invention is defined by the appended claims and relates to absorbent materials from poly(glycerol sebacate). hereinafter referred to as absorbent materials made of PGS, containing an active substance and to a method of its fabrication and uses thereof. In the absorbent material, the active substance, preferably adenosine, is placed during moulding and no subsequent modification/treatment of the material is necessary. The composition of the polymer mixture reduces shrinkage during curing, shortens the curing process and ensures a linear release of the active substance by diffusion.

Poly(glycerol sebacate), abbreviated as PGS , has been known since 2002.[1,2] The beginnings of the use of this polymer in transdermal drug delivery systems can be traced back to 2017.[3] From this group of materials, it is difficult to separate materials that act as dressings, because each of them contains an active substance - either a drug or a healing promoting substance.

In 2017, PGS was used as one of the ingredients of a complex mixture of Chemical compounds being an antibacterial, antioxidant, electroactive hydrogel. Ultimately the product is an injectable dressing for skin wound healing. Polyaniline-modified chitosan and PGS-modified poly(ethylene oxide) were used to produce the dressing.[4] Obtaining such a dressing is multi-step and time-consuming. It also has disadvantages due to the use of substances of natural origin - chitosans - which, due to the way they are obtained and their origin, can differ significantly from each other. For this reason, the solution is not reproducible.

Another option is to produce dressings from PGS and gelatine using electrospinning. Imides are used to crosslink the dressing. Dressings prepared in this way can deliver various active substances such as ciprofloxacin,[5] and antibiotics.[6] In this case, the dressing is based, as well, on a substance of natural origin, what may be disadvantageous for multi-tonnage production.

Another solution, also using electrospinning, is to extrude fibrous dressings from PGS/PHB (PHB - polyhydroxybutyrate) blends. In this solution, the authors chose not to crosslink the produced dressings. Because of this, the dressings degrade very quickly - 20% weight loss within 24 hours and 45% weight loss after 21 days.[7]

Due to such rapid leaching of the polymer from the dressing matrix, it is not suitable for use on open wounds, where it could cause inflammation due to the high availability of free acid groups. Such a dressing is also unable to absorb wound secretions.

Another attempt to produce a fibrous dressing was an electrospinning of PGS, chitin and lignin mixture. With the addition of PGS in the range of 30% and 50%, according to the authors, no fibrous structure was observed on SEM images, the morphology was destructed.[8] This method uses two substances of natural origin, chitin and lignin. Both substances can cause reproducibility problems in multi- tonnage production. Furthermore, no crosslinking process was carried out in this work, making the materials probably not resistant to prolonged exposure to wet environments, such as an open or oozing wound.

Dressings composed almost entirely of synthetic materials were obtained by the A. Khademhosseini's team.[9] In addition to PGS, polycaprolactone was used; unfortunately, chitosan was also a component of the dressing. The authors presented an interesting solution by introducing electronics into the dressing. Unfortunately, they did not use crosslinking of the dressing to harden its structure. As a result, PGS oligomers can leach into the wound environment and lead to an inflammatory reaction. The problem of PGS leaching from a non-woven fabric from polymer blends was solved in 2021 by the use of an additive adhesive cured by light exposure.[10] In addition, the adhesive was intended to ensure good adhesion of the dressing to the skin. However, there is an application problem here. The crosslinking of such a patch should be performed by a specialist who will be able to irradiate the dressing with an appropriate dose of radiation. The patient himself cannot perform the operation of 'sticking' the patch.

A solid PGS-based dressing was obtained in 2016 by adding hyaluronic acid.[11] Unfortunately, as with chitosan and gelatine, hyaluronic acid being of natural origin may have an influence on the subsequent instability of the production of such a dressing. The authors obtained a flexible construct, but chose not to encapsulate any active substance.

An absorbent material comprising a PGS is disclosed in EP 1 448 656. The PGS is cross-linked by heat and comprises an active ingredient.

The work presented here makes it possible to conclude that the most common method of manufacturing dressings based on PGS is electrospinning. In the opinion of the inventors of this application, this method makes it possible to produce interesting fibrous constructs, which, unfortunately, due to their limitations in the form of at least a small thickness, are not suitable for treating wounds with secretions and, due to their delicate structure (low mechanical strength), are not able to provide the patient with free movement. Non-woven fabrics, despite their thinness, do not allow observation of the wound healing process. Visible light is scattered at the edges of the fibres, whereby these materials completely obscure the wound and are opaque. Solid materials often retain a high level of transparency due to their amorphous structure. The ease of their manufacture and the possibility of using industrial techniques to produce them, such as press extrusion, make them materials with high application potential. Fully modified, yet easy to manufacture, the relatively large thickness (1-5 mm) of solid PGS dressings not only provides good strength, but also allows the dressing to be used as a storage matrix for active substances. To the knowledge of the inventors of the present invention, a dressing material fully made of PGS that meets these requirements has not been yet developed. The objective of the present invention is therefore to provide an absorbent material made of PGS, in particular a dressing material, meeting these requirements, and a method of such material's fabrication.

The present invention provides an absorbent material made of PGS, in particular a dressing material, containing an active substance, characterised in that the absorbent material comprises a poly(glycerol sebacate) prepolymer crosslinked with sebacic acid, wherein the sebacic acid is added in the amount that ensures that an equimolar ratio of functional groups is obtained, with the weight error of the poly(glycerol sebacate) prepolymer and the sebacic acid of no more than 2% being acceptable.

Preferably, the degree of crosslinking of the poly(glycerol sebacate) in the absorbent material is not lower than 75 %.

The active substance in the absorbent material may be adenosine.

Ideally, the adenosine content in the absorbent material should not exceed 10 % by weight of the sum of masses of the prepolymer and sebacic acid.

Preferably, the absorbent material according to the invention is sterile.

The invention further provides a method of absorbent material made of PGS fabrication, in particular a dressing material, comprising the following steps:

a) poly(glycerol sebacate) prepolymer and sebacic acid, and optionally the active substance, are mixed;
b) the mixture from step (a) is homogenised to obtain a polymer mixture;
c) if the addition of the active substance is omitted in step (a), the active substance is added to the polymer mixture and the homogenisation process is repeated after the addition of the active substance to the polymer mixture;
d) an absorbent material is formed from the polymer mixture and the formed absorbent material is cured at an elevated temperature;

wherein the sebacic acid in step (a) is used in the amount ensuring that an equimolar ratio of functional groups is maintained, the weight error of the reagents used in step (a) not exceeding 2% being acceptable.

The active substance may be added to the mixture of poly(glycerol sebacate) prepolymer and sebacic acid in step (a) and the whole homogenised, or alternatively, if the addition of the active substance in step (a) is omitted, the active substance may be added to the homogeneous mixture in step (c), whereas the polymer mixture must be homogenised again after the addition of the active substance in step (c).

In the method, a prepolymer obtained by the polycondensation reaction of sebacic acid with glycerol may be used, preferably in the polycondensation reaction a mixture of sebacic acid with glycerol in a molar ratio of 1:2 to 1:5 is heated at a temperature of 130-170°C for 4-8 h, with water stripping and intensive stirring and in an inert gas flow, the mixture is then cooled to a temperature below 30 °C and an ether solvent is added, the volume ratio of the solvent to the reaction mixture being from 5:1 to 20:1, and thereafter the resulting solution is added drop-wise to water at a temperature below 10 °C, with intensive stirring, and after the entire organic solution has been added drop-wise to water, the mixture is cooled to a temperature of -5-5 °C, then filtered under reduced pressure at a temperature of 0-10 °C, after which the product is dried.

The sebacic acid is used in the amount that ensures that an equimolar ratio of functional groups is obtained, with a weighting error of the reagents used of no more than 2% being acceptable.

The amount of sebacic acid required to crosslink one gram of the prepolymer can be calculated using the following formula:

$$m_K[g/g_{of\ prepolymer}] = \frac{500 \times L_{OH}}{56.11} \times 202.25,$$

Preferably, during the homogenisation, the temperature of the mixture does not exceed 50 °C.

Homogenisation can be carried out for 15-30 minutes at least once.

Preferably, homogenisation is carried out at 150-300 rpm.

Adenosine can be used as the active substance.

Preferably, adenosine is added in the amount not exceeding 10 % by weight of the sum of masses of the PGS prepolymer and sebacic acid.

Curing can be carried out in a heated mould, preferably a Teflon mould, at a temperature of 120-300 °C, preferably at the temperature of 200 °C.

Preferably, the heating during curing is carried out for 15-30 min using forced air circulation.

The cured absorbent material can be placed in a packaging.

Preferably, the cured absorbent material, after cooling to room temperature, is placed in a polyamide/polyethylene bag under a vacuum not higher than 0.9 bar and not lower than 0.5 bar.

Preferably, once the absorbent material has been placed in the packaging, it is sterilised.

Ideally, the absorbent material is sterilised by radiation with a dose of 15 to 25 kGy.

The absorbent material made of PGS according to the invention is used in absorbent dressings.

The following example illustrates the invention without limiting it.

## General information

The absorbent material made of PGS was formed in an esterification reaction of an uncrosslinked poly(glycerol sebacate) (PGS) prepolymer with sebacic acid. The prepolymer was produced according to a patented method as described in PL234639.

A homogeniser (a mechanical stirrer suitable for mixing viscous liquids) had to be used to produce the absorbent material made of PGS.

The crosslinking agent, sebacic acid, was added in the amount that ensured that the equimolar ratio of functional groups was obtained. For this purpose, the hydroxyl number ($Z_{\_OH}$) of the poly(glycerol sebacate) prepolymer was determined prior to preparing the polymer mixture according to general knowledge.[12] The amount of sebacic acid ($m_K$) required to crosslink one gram of the prepolymer was then calculated according to the following formula:

$$m_K[g/g_{of\ prepolymer}] = \frac{500 \times L_{OH}}{56.11} \times 202.25,$$

## Manufacture and uses of the absorbent material

In the first step, the weighed prepolymer and the amount of sebacic acid calculated according to the formula above were introduced into the homogeniser. The weighting error should not exceed 2 %. The polymer mixture was homogenised for 15-30 min using 150-300 rpm. If a homogeneous mixture was not obtained, homogenisation had to be repeated. During the homogenisation, the temperature of the mixture should not be raised more than 50°C. In order to shorten the time of this step, sebacic acid as fine-grained as possible, less than 500 pm, should have been used.

In the next step, the polymer mixture obtained was mixed with the active substance (adenosine). The active substance, adenosine, was added in the amount not exceeding 10 % by weight of the sum of masses of the prepolymer and sebacic acid. In the first example, for 100 g of the prepolymer/acid mixture, the amount of adenosine was 10 g. In the second example, for 300 g of the mixture of prepolymer and acid the amount of adenosine was 30 g. The homogenisation step of the polymer mixture with the active substance was carried out in the same way as in the first step.

The next step was to produce the absorbent material by esterification at elevated temperature using a laboratory dryer with forced air circulation in the temperature range of 120-300 °C, preferably at the temperature of 200 °C. The absorbent material was moulded in Teflon moulds, ensuring that the correct shape of the material in relation to the use was obtained. For this purpose, the polymeric mass obtained was poured into a hot mould and then cured at the temperature of 200°C for 15-30 min. The materials obtained were characterised by the degree of crosslinking not lower than 75 %.

The degree of crosslinking should be understood as a percentage of an insoluble (gel) phase to the sum of the insoluble (gel) and soluble (sol) phases.

The absorbent materials were placed in polyamide/polyethylene bags under vacuum after cooling to room temperature. The pressure should not be higher than 0.9 bar and not lower than 0.5 bar.

The product was then sterilised by radiation with a radiation dose of 15 kGy. A dose higher than 25 kGy was not advisable. The absorbent material was ready for use after the manufacture.

Sterile devices should be stored at a temperature not exceeding 5 °C within two years until use, or at a temperature of -20 °C within five years until use.

The absorbent material made PGS is designed for self-use by a patient. After unwrapping, the absorbent material is applied directly to the skin. If adhesion is insufficient, e.g. in the case of profusely oozing wounds, the absorbent material is fixed with a bandage to ensure good gas exchange between the material and the environment.

## References

[1] Y. Wang, B.J. Sheppard, R. Langer, Poly(glycerol sebacate) - A Novel Biodegradable Elastomer for Tissue Engineering, MRS Proc. 724 (2002) N11.1. https://doi.org/10-1557/PROC-724-N11.1.

[2] Y. Wang, G.A. Ameer, B.J. Sheppard, R. Langer, A tough biodegradable elastomer, Nat. Biotechnol. 20 (2002) 602-606. https://doi.org/10.1038/nbt0602-602.

[3] Z. Wu, K. Jin, L. Wang, Y. Fan, A Review: Optimization for Poly(glycerol sebacate) and Fabrication Techniques for Its Centered Scaffolds, Macromol. Biosci. 21 (2021). https://doi.org/10.1002/MABI.202100022.

[4] X. Zhao, FI. Wu, B. Guo, R. Dong, Y. Qiu, P.X.. Ma, Antibacterial anti-oxidant electroactive injectable hydrogel as self-healing wound dressing with haemostasis and adhesiveness for cutaneous wound healing, Biomaterials. 122 (2017) 34-47. https://doi.Org/10.1016/J.BIOMATERIALS.2017.01.011.

[5] S.A. Ayati Najafabadi, P. Shirazaki, A. Zargar Kharazi, J. Varshosaz, M. Tahriri, L. Tayebi, Evaluation of sustained ciprofloxacin release of biodegradable electrospun gelatin/poly(glycerol sebacate) mat membranes for wound dressing applications, Asia-Pacific J. Chem. Eng. 13 (2018). https://doi.org/10.1002/apj.2255.

[6] P. Shirazaki, J. Varshosaz, A.Z. Kharazi, Electrospun Gelatin/poly(Glycerol Sebacate) Membrane with Controlled Release of Antibiotics for Wound Dressing, Adv. Biomed. Res. 6 (2017) 105. https://doi.Org/10.4103/ABR. ABR197_16.

[7] P. Heydari, J. Varshosaz, A. Zargar Kharazi, S. Karbasi, Preparation and evaluation of poly glycerol sebacate/poly hydroxy butyrate core-shell electrospun nanofibers with sequentially release of ciprofloxacin and simvastatin in wound dressings, Polym. Adv. Technol. 29 (2018) 1795-1803. https://doi.org/10.1002/PAT.4286.

[8] T. Abudula, L. Gzara, G. Simonetti, A. Alshahrie, N. Salah, P. Morganti, A. Chianese, A. Fallahi, A. Tamayol, S.A. Bencherif, A. Memic, The Effect of Poly (Glycerol Sebacate) Incorporation within Hybrid Chitin-Lignin Sol-Gel Nanofibrous Scaffolds, Mater. 2018, Vol. 11, Page 451. 11 (2018) 451. https://doi.Org/10.3390/MA11030451.

[9] A. Tamayol, A. Hassani Najafabadi, P. Mostafalu, A.K. Yetisen, M. Commotto, M. Aldhahri, M.S. Abdel-Wahab, Z.I. Najafabadi, S. Latifi, M.Akbari, N. Annabi, S.H. Yun, A. Memic, M.R. Dokmeci, A. Khademhosseini, Biodegradable elastic nanofibrous platforms with integrated flexible heaters for on-demand drug delivery, Sci. Reports 2017 71. 7 (2017) 1-10. https://doi.Org/10.1038/S41598-017-04749-8.

[10] Y.A. Jodat, T. Zhang, Z. Al Tanoury, T. Kamperman, K. Shi, Y. Huang Brigham, H. Adriana Panayi Brigham, H. Yori Endo Brigham, H. Xichi Wang, J. Quint, S. Hassan, J. Lee, A. Flores Huidobro Martinez, S. Lara Ochoa, hiPSC-derived 3D Bioprinted Skeletal Muscle Tissue Implants Regenerate Skeletal Muscle Following Volumetric Muscle Loss, (n.d.). https://doi.Org/10.21203/rs.3.rs-146091/v1.

[11] T.N. Rosenbalm, M. Teruel, C.S. Day, G.L. Donati, M. Morykwas, L. Argenta, N. Kuthirummal, N. Levi-Polyachenko, Structural and mechanical characterisation ot bioresorbable, elastomeric nanocomposites from poly(glycerol sebacate)/nanohydroxyapatite for tissue transport applications, J. Biomed. Mater. Res. Part B Appl. Biomater. 104 (2016) 1366-1373. https://doi.Org/10.1002/JBM.B.33467.

[12] Y.M. Evtushenko, B.E. Zaitsev, V.M. Ivanov, N.A. Khalturinskii, G.Y. Evtushenko, Determination ot Hydroxyl Groups in Epoxy Resins by Potentiometric Titration Using a Reaction with Maleic Anhydride, J. Anal. Chem. 2001 5611. 56 (2001) 1035-1037. https://doi.Org/10.1023/A:1012508809030.

## Claims

1. An absorbent material made of poly(glycerol sebacate) (PGS) in particular a dressing material, containing an active substance, **characterised in that** the material comprises a poly(glycerol sebacate) prepolymer crosslinked by sebacic acid, wherein the sebacic acid is added in the amount that ensures that an equimolar ratio of functional groups is obtained, with the weight error of the poly(glycerol sebacate) prepolymer and the sebacic acid of no more than 2% being acceptable.

2. The material according to claim 1, **characterised in that** the active substance is adenosine.

3. The material according to claim 2, **characterised in that** the adenosine content does not exceed 10 % by weight of the sum of masses of the prepolymer and sebacic acid.

4. The material according to any one of claims 1-3, **characterised in that** it is a sterile material.

5. A method of absorbent material made of PGS fabrication, in particular a dressing material, **characterised in that** it comprises the following steps:

   a) poly(glycerol sebacate) prepolymer and sebacic acid, and optionally the active substance, are mixed;
   b) the mixture from step (a) is homogenised to obtain a polymer mixture;
   c) if the addition of the active substance is omitted in step (a), the active substance is added to the polymer mixture and the homogenisation process is repeated after the addition of the active substance to the polymer mixture;

d) an absorbent material is formed from the polymer mixture and the formed absorbent material is cured at an elevated temperature; wherein

sebacic acid in step (a) is used in the amount ensuring that an equimolar ratio of functional groups is maintained, the weight error of the reagents used in step (a) not exceeding 2 % being acceptable.

6. The method according to claim 5, **characterised in that** in step (a) a prepolymer obtained by polycondensation reaction of sebacic acid with glycerol is used, wherein preferably in the polycondensation reaction a mixture of sebacic acid with glycerol in a molar ratio of 1:2 to 1:5 is heated at a temperature of 130-170 °C for 4-8 h, with water stripping and intensive stirring and in an inert gas flow, the mixture is then cooled to a temperature below 30 °C and an ether solvent is added, the volume ratio of the solvent to the reaction mixture being from 5:1 to 20:1, and thereafter the resulting solution is added drop-wise to water at a temperature below 10°C, with intensive stirring, and after the entire organic solution has been added drop- wise to water, the mixture is cooled to a temperature of -5-5 °C, then filtered under reduced pressure at a temperature of 0-10$^0$ C, and thereafter the product is dried.

7. The method according to claim 6, **characterised in that** the amount of sebacic acid ($m_K$) required to carry out the crosslinking of one gram of prepolymer is calculated according to the following formula:

$$m_K[g/g_{of\ prepolymer}] = \frac{500 \times L_{OH}}{56.11} \times 202.25,$$

after determination of the hydroxyl number $_{(LOH)}$ of the poly(glycerol sebacate) prepolymer.

8. The method according to any one of claims 5-7, **characterised in that** the homogenisation is carried out for 15-30 min at least once.

9. The method according to any one of claims 5-8, **characterised in that** during the homogenisation the temperature of the mixture does not exceed 50°C.

10. The method according to any of the claims 5-9, **characterised in that** the homogenisation is carried out at a frequency of 150-300 rpm.

11. The method according to any one of claims 5-10, **characterised in that** the active substance is adenosine.

12. The method according to claim 11, **characterised in that** adenosine is added in the amount not exceeding 10 % by weight of the sum of masses of the PGS prepolymer and sebacic acid.

13. The method according to any one of claims 5-12, **characterised in that** the curing in step d) is carried out in a heated mould, preferably of a Teflon mould, at a temperature of 120-300°C, preferably at the temperature of 200°C.

14. The method according to claim 13, **characterised in that** the heating is carried out for 15-30 min using forced air circulation.

15. The method according to any of the claims 5-14, **characterised in that** the cured material obtained in step (d) is placed in packagings.

16. Method according to claim 15, **characterised in that** the cured material, after cooling to room temperature, is placed in a polyamide/polyethylene bag under a vacuum not higher than 0.9 bar and not lower than 0.5 bar.

17. Method according to claim 15 or 16 **characterised in that** after the material is placed in the packaging, it is sterilised.

18. Method according to claim 17, **characterised in that** the material is sterilised by radiation with a dose of 15 to 25 kGy.

19. The use of the absorbent material made of PGS as defined in any one of claims 1 to 4 for the preparation of an absorbent dressing.

**Patentansprüche**

1. Ein absorbierendes Material, insbesondere ein Verbandsmaterial, aus Poly(glycerinsebacat) (PGS), enthaltend einen Wirkstoff, **dadurch gekennzeichnet, dass** das Material ein durch Sebacinsäure vernetztes Poly(glycerinsebacat)-Prepolymer umfasst, wobei die Sebacinsäure in einer Menge zugegeben wird, die sicherstellt, dass ein äquimolares Verhältnis von funktionellen Gruppen erhalten wird, wobei ein Gewichtsfehler des Poly(glycerinsebacat)-Prepolymers und der Sebacinsäure von nicht mehr als 2% akzeptabel ist.

2. Das Material nach Anspruch 1, **dadurch gekennzeichnet, dass** der Wirkstoff Adenosin ist.

3. Das Material nach Anspruch 2, **dadurch gekennzeichnet, dass** der Adenosingehalt 10 Gewichts-% der Summe der Massen des Prepolymers und der Sebacinsäure nicht übersteigt.

4. Das Material nach einem der Ansprüche 1-3, **dadurch gekennzeichnet, dass** es ein steriles Material ist.

5. Ein Verfahren eines absorbierenden Materials, insbesondere eines Verbandsmaterials, aus PGS-Herstellung, **dadurch gekennzeichnet, dass** es die folgenden Schritte umfasst:

   a) Poly(glycerinsebacat)-Prepolymer und Sebacinsäure und gegebenenfalls der Wirkstoff gemischt werden;
   b) die Mischung aus Schritt (a) homogenisiert wird, um eine Polymermischung zu erhalten;
   c) falls in Schritt (a) die Zugabe des Wirkstoffs ausgelassen wird, wird der Wirkstoff zu der Polymermischung zugegeben und der Homogenisierungsvorgang wird nach der Zugabe des Wirkstoffs zu der Polymermischung wiederholt;
   d) ein absorbierendes Material aus einer Polymermischung gebildet wird und das gebildete absorbierende Material bei einer erhöhten Temperatur gehärtet wird; wobei Sebacinsäure in Schritt (a) in einer Menge verwendet wird, die sicherstellt, dass ein äquimolares Verhältnis von funktionellen Gruppen aufrechterhalten wird, wobei der Gewichtsfehler der in Schritt (a) verwendeten Reagenzien, der 2% nicht übersteigt, akzeptabel ist.

6. Das Verfahren nach Anspruch 5, **dadurch gekennzeichnet, dass** in Schritt (a) ein durch Polykondensationsreaktion von Sebacinsäure mit Glycerin erhaltenes Prepolymer verwendet wird, wobei vorzugsweise in der Polykondensationsreaktion eine Mischung von Sebacinsäure mit Glycerin in einem Molverhältnis von 1:2 bis 1:5 unter Wasserstrippen und intensivem Rühren und in einem Inertgasstrom für 4-8 h bei einer Temperatur von 130-170°C erwärmt wird, die Mischung dann auf eine Temperatur unter 30°C abgekühlt wird und ein Etherlösungsmittel zugegeben wird, wobei das Volumenverhältnis des Lösungsmittels zu der Reaktionsmischung von 5:1 bis 20:1 beträgt, und danach die resultierende Lösung unter intensivem Rühren tropfenweise zu Wasser bei einer Temperatur unter 10°C zugegeben wird, und nachdem die gesamte organische Lösung tropfenweise zu Wasser zugegeben wurde, die Mischung auf eine Temperatur von -5-5°C abgekühlt wird, dann unter vermindertem Druck bei einer Temperatur von 0-100°C filtriert wird und danach das Produkt getrocknet wird.

7. Das Verfahren nach Anspruch 6, **dadurch gekennzeichnet, dass** die Menge an Sebacinsäure (mK), die erforderlich ist, um die Vernetzung von einem Gramm des Prepolymers durchzuführen, nach der folgenden Formel berechnet wird: nach der Bestimmung der Hydroxylzahl (LOH) des Poly(glycerinsebacat)-Prepolymers.

$$m_K[g/g_{des\ Prepolymers}] = \frac{500 \times L_{OH}}{56.11} \times 202.25,$$

8. Das Verfahren nach einem der Ansprüche 5-7, **dadurch gekennzeichnet, dass** die Homogenisierung mindestens einmal für 15-30 min durchgeführt wird.

9. Das Verfahren nach einem der Ansprüche 5-8, **dadurch gekennzeichnet, dass** während der Homogenisierung die Temperatur der Mischung 50°C nicht übersteigt.

10. Das Verfahren nach einem der Ansprüche 5-9, **dadurch gekennzeichnet, dass** die Homogenisierung bei einer Frequenz von 150-300 U/min durchgeführt wird.

11. Das Verfahren nach einem der Ansprüche 5-10, **dadurch gekennzeichnet, dass** der Wirkstoff Adenosin ist.

**12.** Das Verfahren nach Anspruch 11, **dadurch gekennzeichnet, dass** Adenosin in einer Menge zugegeben wird, die 10 Gewichts-% der Summe der Massen des PGS-Prepolymers und der Sebacinsäure nicht übersteigt.

**13.** Das Verfahren nach einem der Ansprüche 5-12, **dadurch gekennzeichnet, dass** das Härten in Schritt d) in einer erwärmten Form, vorzugsweise einer Teflonform, bei einer Temperatur von 120-300°C, vorzugsweise bei der Temperatur von 200°C, durchgeführt wird.

**14.** Das Verfahren nach Anspruch 13, **dadurch gekennzeichnet, dass** die Erwärmung für 15-30 min unter Verwendung einer erzwungenen Luftzirkulation durchgeführt wird.

**15.** Das Verfahren nach einem der Ansprüche 5-14, **dadurch gekennzeichnet, dass** das in Schritt (d) erhaltene gehärtete Material in Verpackungen platziert wird.

**16.** Verfahren nach Anspruch 15, **dadurch gekennzeichnet, dass** das gehärtete Material nach dem Abkühlen auf Raumtemperatur in einen Polyamid/Polyethylen-Beutel unter einem Vakuum von nicht höher als 0,9 bar und nicht niedriger als 0,5 bar platziert wird.

**17.** Verfahren nach Anspruch 15 oder 16, **dadurch gekennzeichnet, dass** nach dem Platzieren des Materials in die Verpackung es sterilisiert wird.

**18.** Verfahren nach Anspruch 17, **dadurch gekennzeichnet, dass** das Material durch Bestrahlung mit einer Dosis von 15 bis 25 kGy sterilisiert wird.

**19.** Die Verwendung des absorbierenden Materials aus PGS wie in Ansprüchen 1 bis 4 definiert zur Vorbereitung eines absorbierenden Verbands.

**Revendications**

**1.** Matériau absorbant constitué de sébacate de polyglycérol (PGS), en particulier un matériau de pansement, contenant une substance active, **caractérisé en ce que** le matériau comprend un prépolymère de sébacate de polyglycérol réticulé par l'acide sébacique, l'acide sébacique étant ajouté en quantité assurant qu'un rapport équimolaire des groupes fonctionnels est obtenu, avec une erreur de pondération du prépolymère de sébacate de polyglycérol et d'acide sébacique d'un maximum de 2 % étant acceptable.

**2.** Matériau selon la revendication 1, **caractérisé en ce que** la substance active est l'adénosine.

**3.** Matériau selon la revendication 2, **caractérisé en ce que** le contient de l'adénosine ne dépasse pas 10% en poids de la somme des masses du prépolymère et d'acide sébacique.

**4.** Matériau selon l'une quelconque des revendications 1-3, **caractérisé en ce qu'**il est un matériau stérile.

**5.** Procédé de fabrication d'un matériau absorbant constitué de sébacate de polyglycérol (PGS), en particulier un matériau de pansement, **caractérisé en ce qu'**il comprend les étapes suivantes :

a) le prépolymère de sébacate de polyglycérol et l'acide sébacique, et éventuellement la substance active, sont mélangés ;
b) le mélange issu d'étape (a) est homogénéisé pour obtenir un mélange polymère ;
c) lorsque l'ajout de la substance active est omis à l'étape (a), la substance active est ajoutée au mélange polymère et le procédé d'homogénéisation est répétée à la suite d'ajout de la substance active au mélange polymère ;
d) un matériau absorbant est formé à partir du mélange polymère et le matériau absorbant formé est durci à une température élevée ; dans laquelle
l'acide sébacique à l'étape (a) est utilisé en quantité assurant qu'un rapport équimolaire des groupes fonctionnels est conservé, l'erreur de pondération des réactifs utilisés à l'étape (a) ne dépassant pas 2% étant acceptable.

**6.** Procédé selon la revendication 5, **caractérisé en ce qu'**à l'étape (a) on utilise un prépolymère obtenu par réaction de polycondensation d'acide sébacique avec glycérol, avantageusement dans la réaction de polycondensation un mélange d'acide sébacique avec glycérol à un rapport molaire de 1:2 à 1:5 étant chauffé à une température de 130-170°C pendant 4-8 heures, par stripage à l'eau et agitation puissante et dans un flux du gaz inerte, le mélange est ensuite refroidi à une température inférieure à 30°C et un solvant éther est ajouté, le rapport volumique du solvant par rapport au mélange réactionnel étant de 5 :1 à 20 :1, et ultérieurement la solution résultante est ajoutée sous forme de gouttelettes à l'eau à une température inférieure à 10°C, par agitation puissante, et après l'ensemble de solution organique a été ajouté sous forme des gouttelettes à l'eau, le mélange est refroidi à une température de -5-5°C, filtré sous pression réduite à une température de 0-10°C, et ultérieurement le produit est séché.

7. Procédé selon la revendication 6, **caractérisé en ce que** la quantité d'acide sébacique ($m_k$) requise pour effectuer la réticulation d'un gramme du prépolymère est calculée selon la formule suivante :

$$m_k \left[ {}^g\!/\!_{g_{prépolymère}} \right] = \frac{500 \times L_{OH}}{56.11} + 202.25,$$

à la suite de la détermination d'indice d'hydroxyle ($L_{OH}$) du prépolymère de sébacate de polyglycérol.

8. Procédé selon l'une quelconque des revendications 5-7, **caractérisé en ce que** l'homogénéisation est effectuée pendant 15-30 minutes au moins une fois.

9. Procédé selon l'une quelconque des revendications 5-8, **caractérisé en ce que** lors de l'homogénéisation la température du mélange ne dépasse pas 50°C.

10. Procédé selon l'une quelconque des revendications 5-9, **caractérisé en ce que** l'homogénéisation est effectuée à une fréquence de 150-300 tours par minute.

11. Procédé selon l'une quelconque des revendications 5-10, **caractérisé en ce que** la substance active est l'adénosine.

12. Procédé selon la revendication 11, **caractérisé en ce que** l'adénosine est ajoutée en quantité ne dépassant pas 10% en poids de la somme des masses du prépolymère PGS et d'acide sébacique.

13. Procédé selon l'une quelconque des revendications 5-12, **caractérisé en ce que** le durcissement à l'étape d) est effectué dans un moule à chauffage, avantageusement du moule téflon, à une température de 120-300°C, avantageusement à la température de 200°C.

14. Procédé selon la revendication 13, **caractérisé en ce que** le chauffage est effectué pendant 15-30 minutes en utilisant la circulation d'air forcée.

15. Procédé selon l'une quelconque des revendications 5-14, **caractérisé en ce que** le matériau durci obtenu à l'étape (d) est conditionné dans des emballages.

16. Procédé selon la revendication 15, **caractérisé en ce que** le matériau durci, suivant le refroidissement à la température ambiante, est conditionné dans un sac en polyamide/polyéthylène sous vide non supérieure à 0.9 bar et non inférieure à 0.5 bar.

17. Procédé selon la revendication 15 ou 16, **caractérisé en ce qu'**après le matériau est conditionné dans l'emballage, il est stérilisé.

18. Procédé selon la revendication 17, **caractérisé en ce que** le matériau est stérilisé par rayonnement à une dose de 15 à 25 kGy.

19. Utilisation du matériau absorbant constitué de PGS telle que définie selon l'une quelconque des revendications 1 à 4 pour préparation d'un pansement absorbant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1448656 A

- PL 234639

**Non-patent literature cited in the description**

- **Y. WANG** ; **B.J. SHEPPARD** ; **R. LANGER**. Poly(glycerol sebacate) - A Novel Biodegradable Elastomer for Tissue Engineering. *MRS Proc.*, 2002, vol. 724, https://doi.org/10-1557/PROC-724-N11.1
- **Y. WANG** ; **G.A. AMEER** ; **B.J. SHEPPARD** ; **R. LANGER**. A tough biodegradable elastomer. *Nat. Biotechnol.*, 2002, vol. 20, 602-606, https://doi.org/10.1038/nbt0602-602
- **Z. WU** ; **K. JIN** ; **L. WANG** ; **Y. FAN**. A Review: Optimization for Poly(glycerol sebacate) and Fabrication Techniques for Its Centered Scaffolds. *Macromol. Biosci.*, 2021, vol. 21, https://doi.org/10.1002/-MABI.202100022
- **X. ZHAO** ; **FI. WU** ; **B. GUO** ; **R. DONG** ; **Y. QIU** ; **P.X.. MA**. Antibacterial anti-oxidant electroactive injectable hydrogel as self-healing wound dressing with haemostasis and adhesiveness for cutaneous wound healing. *Biomaterials*, 2017, vol. 122, 34-47, https://doi.Org/10.1016/J.BIOMATER-IALS.2017.01.011.
- **A. TAMAYOL** ; **A. HASSANI NAJAFABADI** ; **P. MOSTAFALU** ; **A.K. YETISEN** ; **M. COMMOTTO** ; **M. ALDHAHRI** ; **M.S. ABDEL-WAHAB** ; **Z.I. NAJAFABADI** ; **S. LATIFI** ; **M. AKBARI**. Biodegradable elastic nanofibrous platforms with integrated flexible heaters for on-demand drug delivery. *Sci. Reports*, 2017, vol. 7 (2017), 1-10, https://doi.Org/10.1038/S41598-017-04749-8

- **Y.A. JODAT** ; **T. ZHANG** ; **Z. AL TANOURY** ; **T. KAMPERMAN** ; **K. SHI** ; **Y. HUANG BRIGHAM** ; **H. ADRIANA PANAYI BRIGHAM** ; **H. YORI ENDO BRIGHAM** ; **H. XICHI WANG** ; **J. QUINT**. *hiPSC-derived 3D Bioprinted Skeletal Muscle Tissue Implants Regenerate Skeletal Muscle Following Volumetric Muscle Loss*, https://doi.Org/10.21203/rs.3.rs-146091/v1
- **T.N. ROSENBALM** ; **M. TERUEL** ; **C.S. DAY** ; **G.L. DONATI** ; **M. MORYKWAS** ; **L. ARGENTA** ; **N. KUTHIRUMMAL** ; **N. LEVI-POLYACHENKO**. Structural and mechanical characterisation ot bioresorbable, elastomeric nanocomposites from poly(glycerol sebacate)/nanohydroxyapatite for tissue transport applications. *J. Biomed. Mater. Res. Part B Appl. Biomater.*, 2016, vol. 104, 1366-1373, https://doi.Org/10.1002/JBM.B.33467
- **Y.M. EVTUSHENKO** ; **B.E. ZAITSEV** ; **V.M. IVANOV** ; **N.A. KHALTURINSKII** ; **G.Y. EVTUSHENKO**. Determination ot Hydroxyl Groups in Epoxy Resins by Potentiometric Titration Using a Reaction with Maleic Anhydride. *J. Anal. Chem.*, 2001, vol. 56 (2001), 1035-1037, https://doi.Org/10.1023/A:1012508809030